# EUROPÄISCHE PATENTSCHRIFT

(11) **EP 4 660 374 B1**
(45) Veröffentlichungstag und Bekanntmachung des Hinweises auf die Patenterteilung: **13.05.2026**
(21) Anmeldenummer: 25176542.6
(22) Anmeldetag: 15.05.2025
(51) Int. Cl.: E01C 19/26, E02D 3/026

(54) **BODENVERDICHTER**
FLOOR COMPRESSOR
COMPACTEUR DE SOL

(30) Priorität: 05.06.2024 DE 102024115572
(43) Veröffentlichungstag der Anmeldung: 10.12.2025
(73) Patentinhaber: Hamm AG, 95643 Tirschenreuth (DE)
(72) Erfinder: Mühlhausen, Axel, Kassel (DE); Krockauer, Rainer, Plößberg (DE); Kreger, Marco, Tirschenreuth (DE); Meindl, Klaus, Bärnau/Hohenthan (DE)
(74) Vertreter: Ruttensperger Lachnit Trossin Gomoll

(56) Entgegenhaltungen:
- EP-A1- 3 130 939
- WO-A1-2021/229146

## Beschreibung

Die vorliegende Erfindung betrifft einen Bodenverdichter, welcher dazu eingesetzt werden kann, Bodenmaterial, wie zum Beispiel Asphalt, zu verdichten.

Aus der WO 2021/229146 A1 ist ein als sogenannter Walzenzug ausgebildeter Bodenverdichter bekannt, bei welchem ein Verdichterrahmen einen Hauptrahmen mit einem daran vorgesehenen Antriebsaggregat, einem Bedienstand und durch das Antriebsaggregat angetriebenen Antriebsrädern umfasst. Mit dem Hauptrahmen ist ein Nebenrahmen um eine im Wesentlichen vertikal orientierte Schwenkachse schwenkbar verbunden. An dem Nebenrahmen ist eine Verdichterwalze um eine im Wesentlichen in einer Bodenverdichter-Querrichtung und zu einer Bodenverdichter-Längsrichtung im Wesentlichen orthogonal sich erstreckende Walzendrehachse drehbar getragen. An einer Unterseite des Hauptrahmens ist in vertikaler Richtung nach unten hervorstehend eine Bodenradar-Feuchtigkeitsmessanordnung derart getragen, dass eine Antenne dieser Bodenradar-Feuchtigkeitsmessanordnung in geringem Abstand zu dem hinsichtlich seines Feuchtigkeitsgehalt zu erfassenden Boden positioniert ist.

Es ist die Aufgabe der vorliegenden Erfindung, einen Bodenverdichter mit einer gegen äußere Einflüsse geschützten Bodenradar-Dichtemessanordnung vorzusehen.

Erfindungsgemäß wird diese Aufgabe gelöst durch einen Bodenverdichter, umfassend wenigstens eine an einem Verdichterrahmen drehbar getragene Verdichterwalze und wenigstens eine in einem Innenvolumenbereich des Verdichterrahmens angeordnete Bodenradar-Dichtemessanordnung.

Durch das Anordnen wenigstens einer Bodenradar-Dichtemessanordnung in einem Innenvolumenbereich des Verdichterrahmens, also nicht am Verdichterrahmen nach außen hervorstehend, ist eine derartige Bodenradar-Dichtemessanordnung gegen physische Einwirkung von außen im Wesentlichen geschützt am Bodenverdichter getragen. Da in Bereichen, in welchen derartige Bodenverdichter arbeiten, häufig Hindernisse vorhanden sind, welche mit dem Bodenverdichter kollidieren können, kann eine Beschädigung einer derart untergebrachten Bodenradar-Dichtemessanordnung durch ein mit dem Bodenverdichter kollidierendes Hindernis vermieden werden. Auch bei Reinigungsarbeiten beispielsweise vermittels eines Hochdruckreinigers besteht nicht die Gefahr einer Beschädigung einer am Bodenverdichter untergebrachten Bodenradar-Dichtemessanordnung.

Der Verdichterrahmen kann einen Hauptrahmen und wenigstens einen mit dem Hauptrahmen um eine Lenkachse schwenkbar verbundenen Nebenrahmen umfassen, wobei an dem Hauptrahmen ein zum Antreiben des Bodenverdichters zu betreibendes Antriebsaggregat vorgesehen ist und an dem wenigstens einen Nebenrahmen eine Verdichterwalze drehbar getragen ist.

Ein derartiger Bodenverdichter kann als sogenannte Tandemwalze mit einer an dem Hauptrahmen drehbar getragenen Verdichterwalze und einer an einem Nebenrahmen drehbar getragenen Verdichterwalze, als sogenannter Walzenzug mit einer an dem Nebenrahmen drehbar getragenen Verdichterwalze und an dem Hauptrahmen getragenen Antriebsrädern ausgebildet sein, oder kann als sogenannter schemelgelenkter Bodenverdichter ausgebildet sein, bei welchem am Hauptrahmen zwei als Lenkschemel ausgebildeten Nebenrahmen schwenkbar angebracht sind und an jedem Nebenrahmen eine Verdichterwalze drehbar getragen ist.

Ferner können bei einem derartigen Bodenverdichter unabhängig von dessen Bauart eine oder mehrere Verdichterwalzen am Hauptrahmen oder/und an wenigstens einem Nebenrahmen als in Richtung einer Walzendrehachse durchgehend oder geteilt ausgebildete Verdichterwalze mit einem im Allgemeinen aus Stahlmaterial ausgebildeten Walzenmantel aufgebaut sein. Alternativ oder zusätzlich können bei einem derartigen Bodenverdichter eine oder mehrere Verdichterwalzen jeweils mit einer Mehrzahl von in Richtung einer Walzendrehachse aufeinander folgend angeordneten Gummiradwalzen ausgebildet sein.

Für die schwenkbare Verbindung kann der wenigstens eine Nebenrahmen einen in einer Bodenverdichter-Querrichtung sich erstreckenden Querträger umfassen, wobei der Nebenrahmen im Bereich des Querträgers mit dem Hauptrahmen schwenkbar verbunden ist.

In einem Nebenrahmen-Innenvolumenbereich des Querträgers kann wenigstens eine Bodenradar-Dichtemessanordnung angeordnet sein. Hierzu kann der Querträger zum Bereitstellen eines Nebenrahmen-Innenvolumenbereichs als Hohlträger bzw. Hohlprofilträger ausgebildet sein.

Insbesondere bei Ausgestaltung des Bodenverdichters als Tandemwalze oder Walzenzug kann der wenigstens eine Nebenrahmen einen in einer Bodenverdichter-Längsrichtung in Abstand zu dem Querträger angeordneten und mit dem Querträger durch zwei in der Bodenverdichter-Querrichtung in Abstand zueinander angeordnete und im Wesentlichen in der Bodenverdichter-Längsrichtung sich erstreckende Längsträger verbundenen, im Wesentlichen in der Bodenverdichter-Querrichtung sich erstreckenden weiteren Querträger umfassen, wobei die Verdichterwalze an den Längsträgern drehbar getragen ist. In einem Nebenrahmen-Innenvolumenbereich des weiteren Querträgers oder/und in einem Nebenrahmen-Innenvolumenbereich von wenigstens einem Längsträger kann wenigstens eine Bodenradar-Dichtemessanordnung angeordnet sein.

Auch hierzu kann vorgesehen sein, dass der weitere Querträger oder/und wenigstens einer der Längsträger zum Bereitstellen eines Nebenrahmen-Innenvolumenbereichs als Hohlträger bzw. Hohlprofilträger ausgebildet ist.

Wenn der Bodenverdichter zwei als Lenkschemel ausgebildete Nebenrahmen umfasst, die mit dem Hauptrahmen um eine jeweilige Lenkachse schwenkbar verbunden sind, kann in einem Nebenrahmen-Innenvolumenbereich von wenigstens einem der als Lenkschemel ausgebildeten Nebenrahmen wenigstens eine Bodenradar-Dichtemessanordnung angeordnet sein.

Bei einer weiteren Ausgestaltungsart des erfindungsgemäßen Bodenverdichters kann der Hauptrahmen einen das Antriebsaggregat und wenigstens eine Bodenradar-Dichtemessanordnung enthaltenden Hauptrahmen-Innenvolumenbereich aufweisen.

Zur Durchführung von Wartungsarbeiten an dem Antriebsaggregat oder in Verbindung mit diesem am Hauptrahmen getragenen Zusatzaggregaten kann in dem Hauptrahmen wenigstens eine vorzugsweise verschließbare Revisionsöffnung vorgesehen sein. Dabei kann wenigstens eine Bodenradar-Dichtemessanordnung derart positioniert sein, dass auf diese, vorzugsweise jede in dem Hauptrahmen-Innenvolumenbereich angeordnete Bodenradar-Dichtemessanordnung, über wenigstens eine Revisionsöffnung Zugriff besteht. Dies bedeutet, dass eine derart positionierte Bodenradar-Dichtemessanordnung durch die Revisionsöffnung hindurch gewartet und erforderlichenfalls ersetzt werden kann.

Um eine gegen Vibrationen geschützte Unterbringung einer oder mehrerer Bodenradar-Dichtemessanordnung in an dem Verdichterrahmen des Bodenverdichters zu gewährleisten, wird vorgeschlagen, dass wenigstens eine, vorzugsweise jede Bodenradar-Dichtemessanordnung einen vermittels wenigstens eines elastisch verformbaren Aufhängungselements an dem Verdichterrahmen getragenen Sensorträger und wenigstens einen an dem Sensorträger getragenen Bodenradar-Dichtesensor umfasst.

Für eine stabile Halterung am Verdichterrahmen kann wenigstens eine, vorzugsweise jede Bodenradar-Dichtemessanordnung an einem plattenartigen Trägerbereich des Verdichterrahmens getragen sein.

Die Erfassung des unter bzw. im Bereich eines Bodenverdichters liegenden Bodens durch eine in einem Innenvolumenbereich des Bodenverdichters, also einem Nebenrahmen-Innenvolumenbereich N_{VI} oder/und einem Hauptrahmen-Innenvolumenbereich H_{VI}, angeordnete Bodenradar-Dichtemessanordnung kann dadurch ermöglicht werden, dass an dem Verdichterrahmen in Zuordnung zu wenigstens einer, vorzugsweise jeder Bodenradar-Dichtemessanordnung eine Messöffnung vorgesehen ist.

Um einen durch den Bodenverdichter generierten Verdichtungszustand eines zu verdichtenden Bodens umfangreich erfassen zu können, wird vorgeschlagen, dass in Zuordnung zu wenigstens einer, vorzugsweise jeder Verdichterwalze in einer Bodenverdichter-Längsrichtung an jeder Seite der Verdichterwalze wenigstens eine Bodenradar-Dichtemessanordnung vorgesehen ist, oder/und dass wenigstens zwei Bodenradar-Dichtemessanordnungen in einer Bodenverdichter-Querrichtung in Abstand zueinander angeordnet sind.

Die vorliegende Erfindung wird nachfolgend mit Bezug auf die beiliegenden Figuren detailliert beschrieben. Es zeigt:
- Fig. 1: in prinzipartiger Darstellung eine Draufsicht auf einen als Tandemwalze ausgebildeten Bodenverdichter;
- Fig. 2: in prinzipartiger Darstellung eine in einem Innenvolumenbereich des Bodenverdichters angeordnete Bodenradar-Dichtemessanordnung;
- Fig. 3: eine Seitenansicht eines als Walzenzug ausgebildeten Bodenverdichters;
- Fig. 4: eine Seitenansicht einer als Tandemwalze ausgebildeten Kleinwalze;
- Fig. 5: eine prinzipartige Seitenansicht eines schemelgelenkten Bodenverdichters.

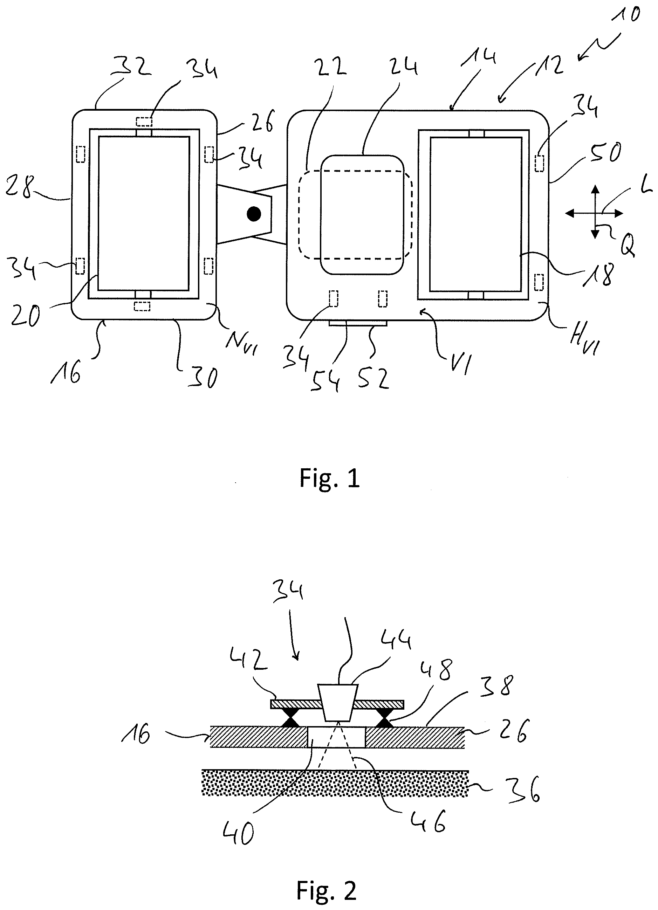

Der in Fig 1 dargestellte Bodenverdichter 10 umfasst einen allgemein mit 12 bezeichneten Verdichterrahmen mit einem Hauptrahmen 14 und einem mit dem Hauptrahmen 14 um eine zur Zeichenebene der Fig. 1 im Wesentlichen orthogonal stehende Lenkachse A schwenkbar verbundenen Nebenrahmen 16. Am Hauptrahmen 14 ist eine Verdichterwalze 18 um eine in einer Bodenverdichter-Querrichtung Q sich erstreckende und zu einer Bodenverdichter-Längsrichtung L im Wesentlichen orthogonale Walzendrehachse drehbar getragen. Am Nebenrahmen 16 ist eine Verdichterwalze 20 um eine Walzenachse drehbar getragen, welche sich gleichermaßen im Wesentlichen in der Bodenverdichter-Querrichtung Q bzw. orthogonal zur Bodenverdichter-Längsrichtung L erstreckt.

Am Hauptrahmen 14 ist ein beispielsweise mit einer Brennkraftmaschine oder einem Elektromotor ausgebildetes Antriebsaggregat 22 getragen, welches die Antriebsenergie zum Betreiben des Bodenverdichters 10 bereitstellt. Ferner ist am Hauptrahmen 14 ein Bedienstand 24 für eine den Bodenverdichter 10 bedienende Bedienperson vorgesehen.

Der Nebenrahmen 16 umfasst einen im Wesentlichen in der Bodenverdichter-Querrichtung Q sich erstreckenden Querträger 26, über welchen der Nebenrahmen 16 mit dem Hauptrahmen 14 schwenkbar verbunden ist. Ein weiterer Querträger 28 ist in der Bodenverdichter-Längsrichtung L in Abstand zu dem Querträger 26 und zu diesen im Wesentlichen parallel sich erstreckend angeordnet und ist mit dem Querträger 26 durch zwei im Wesentlichen in der Bodenverdichter-Längsrichtung L sich erstreckende und in der Bodenverdichter-Querrichtung Q in Abstand zueinander angeordnete Längsträger 30, 32 verbunden, so dass der Nebenrahmen 16 die an den Längsträgern 30, 32 desselben drehbar getragene Verdichterwalze 20 im Wesentlichen vollständig umgibt. Zumindest einer der beiden Querträger 26, 28 oder/und zumindest einer der Längsträger 30, 32 ist als Hohlträger ausgebildet, und stellt somit einen Nebenrahmen-Innenvolumenbereich N_{VI} bereit, in welchem zumindest eine Bodenradar-Dichtemessanordnung 34 angeordnet ist. Vermittels einer derartigen Bodenradar-Dichtemessanordnung 34 kann durch Abtastung des von dem Bodenverdichter 10 überfahrenen Bodens bzw. Untergrunds dessen Verdichtungszustand bzw. Dichte repräsentierende Informationen generiert und zur weiteren Auswertung zur Verfügung gestellt werden.

Eine derartige beispielsweise am Querträger 26 des Nebenrahmens 16 angeordnete Bodenradar-Dichtemessanordnung 34 ist in Fig. 2 veranschaulicht. In Zuordnung zu dieser Bodenradar-Dichtemessanordnung 34 ist an einem dem zu verdichtenden bzw. verdichteten Boden 36 zugewandten, plattenartigen Bereich 38 des als Hohlträger ausgebildeten Querträgers 26 eine Messöffnung 40 gebildet, durch welche hindurch erfassungstechnischer Zugriff auf den unter diesem Bereich des Nebenrahmens 16 liegenden Abschnitt des Bodens 36 besteht. Die Bodenradar-Dichtemessanordnung 34 umfasst einen beispielsweise plattenartigen Sensorträger 42, an welchem im dargestellten Ausgestaltungsbeispiel ein Bodenradar-Dichtesensor 44 derart getragen ist, dass dessen Messbeobachtungsfeld 46 durch die Messöffnung 40 hindurch nach unten auf den Boden 36 gerichtet ist. Um den Bodenradar-Dichtesensor 44 gegen Vibrationen oder sonstige Erschütterungen zu schützen, ist der Sensorträger 42 über mehrere elastische, beispielsweise mit Gummimaterial aufgebaute Aufhängungselemente 48 an dem Querträger 26 bzw. dem plattenartigen Bereich 38 desselben getragen.

Um insbesondere die durch die Verdichterwalze 20 generierte Veränderung im Verdichtungszustand des Bodens 16 erfassen zu können, ist es vorteilhaft, in der Bodenverdichter-Längsrichtung L beidseits der Verdichterwalze 20 jeweils wenigstens eine derartige Bodenradar-Dichtemessanordnung 34 vorzusehen. Dies bedeutet, dass auch in dem zum Hauptrahmen 14 in größerem Abstand liegenden weiteren Querträger 28 eine derartige Bodenradar-Dichtemessanordnung 34 vorgesehen sein kann, beispielsweise in der in Fig. 2 dargestellten Art und Weise.

Um in der Breitenrichtung, also der Verdichter-Querrichtung Q, des überfahrenen Bodens Information über den Verdichtungszustand bereitstellen zu können, ist es weiter vorteilhaft, in der Verdichter-Querrichtung Q in Abstand zueinander liegende Bodenradar-Dichtemessanordnungen 34 beispielsweise am Querträger 26 oder/und am weiteren Querträger 28 bereitzustellen.

Alternativ oder zusätzlich zum Bereitstellen einer oder mehrere Bodenradar-Dichtemessanordnungen 34 in einem oder in beiden Querträger 26, 28 können insbesondere bei Ausgestaltung der Längsträger 30, 32 als Hohlträger auch darin eine oder mehrere Dichtemessanordnungen vorgesehen sein.

Alternativ oder zusätzlich zum Vorsehen einer oder mehrerer Bodenradar-Dichtemessanordnungen 34 am Nebenrahmen 16, können auch am Hauptrahmen 14 eine oder mehrere derartige Bodenradar-Dichtemessanordnungen 34 vorgesehen sein. Beispielsweise ist es möglich, eine oder mehrere Bodenradar-Dichtemessanordnungen 34 in einem Querträger 50 des Hauptrahmens 40 unterzubringen. Besonders vorteilhaft ist das Vorsehen derartiger Bodenradar-Messanordnungen 34 im Bereich einer durch ein plattenartiges Verschlusselement 52 abschließbaren Revisionsöffnung 54 des Hauptrahmens 14 anzuordnen, durch welche hindurch Zugriff auf das Antriebsaggregat 22 oder andere in einem Hauptrahmen-Innenvolumenbereich H_{VI} untergebrachte Aggregate besteht. Beispielsweise können derartige im Bereich der Revisionsöffnung 54 vorgesehene Bodenradar-Dichtemessanordnungen 34 an einer Bodenplatte des Hauptrahmens 14 in einer der Fig. 2 entsprechenden Art und Weise dem zu verdichtenden bzw. verdichteten Boden gegenüberliegend positioniert werden.

Durch die Unterbringung einer oder mehrerer Bodenradar-Dichtemessanordnungen 34 in einem Innenvolumenbereich VI des Bodenverdichters 10, also einem Nebenrahmen-Innenvolumenbereich N_{VI} oder/und einem Hauptrahmen-Innenvolumenbereich H_{VI}, ist eine gegen äußere Einflüsse geschützte Unterbringung gewährleistet, welche insbesondere aufgrund der in Fig. 2 dargestellten Positionierung derartiger Bodenradar-Dichtemessanordnungen 34 gleichwohl eine zuverlässige, exakte Erfassung des Zustandes des Bodens gewährleistet.

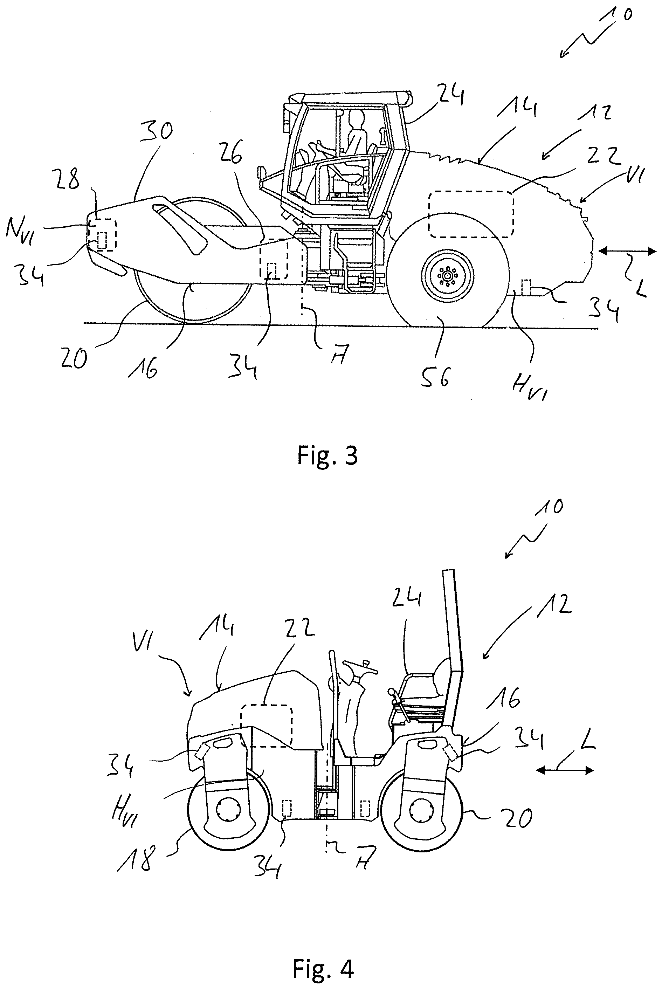

Die Fig. 3 zeigt einen als Walzenzug ausgebildeten Bodenverdichter 10 mit einem allgemein auch als Hinterwagen bezeichneten Hauptrahmen 14 und einem allgemein als Vorderwagen bezeichneten Nebenrahmen 16. Diese sind um eine Lenkachse A schwenkbar miteinander verbunden. Am Hauptrahmen 14 sind das Antriebsaggregat 22 und der Bedienstand 24 untergebracht. Ferner sind am Hauptrahmen 14 zwei durch das Antriebsaggregat 22 zur Drehung und somit auch zum Voranbewegen des Bodenverdichters 10 antreibbare Antriebsräder 56 vorgesehen.

Der Nebenrahmen weist die beiden Querträger 26, 28 und die diese verbindenden Längsträger 30, 32 auf, welche die am Nebenrahmen 16 drehbar getragene Verdichterwalze 20 umgeben.

Wie in Fig. 3 veranschaulicht, können beispielsweise in einem oder beiden der Querträger 26, 28 eine oder mehrere Bodenradar-Dichtemessanordnungen 34 in der vorangehend mit Bezug auf die Fig. 2 beschriebene Art und Weise untergebracht sein. Bei Ausgestaltung der Längsträger 30, 32 als Hohlträger können alternativ oder zusätzlich auch darin eine oder mehrere Bodenradar-Dichtemessanordnungen 34 untergebracht sein. Auch am Hauptrahmen 14 können beispielsweise in einem Heckbereich desselben eine oder mehrere Bodenradar-Dichtemessanordnungen 34 angeordnet sein, oder können in der Bodenverdichter-Längsrichtung L in Abstand zueinander positionierte Bodenradar-Dichtemessanordnungen 34 vorgesehen sein.

Eine weitere Ausgestaltungsart eines Bodenverdichters 10 ist in Fig. 4 dargestellt. Der Bodenverdichter 10 der Fig. 4 ist grundsätzlich als Tandemwalze mit zwei Verdichterwalzen 18, 20 ausgebildet. Die Verdichterwalze 20 ist an einem auch das Antriebsaggregat 32 tragenden Hauptrahmen 14 getragen. Die Verdichterwalze 20 ist an dem den Bedienstand 24 aufweisenden Nebenrahmen 16 getragen. Der Hauptrahmen 14 und der Nebenrahmen 16 sind um die Lenkachse A schwenkbar bezüglich einander getragen. Am Hauptrahmen 14 oder/und am Nebenrahmen 16 können eine oder mehrere Bodenradar-Dichtemessanordnungen 34 beispielsweise an einer jeweiligen Bodenplatte in der in Fig. 2 dargestellten Art und Weise getragen sein. Auch ist es möglich, beispielsweise am Hauptrahmen 14 eine oder mehrere Bodenradar-Dichtemessanordnung 34 im Hauptrahmen-Innenvolumenbereich H_{VI} so zu positionieren, dass diese schräg nach vorne auf den vor der Verdichterwalze 18 des Hauptrahmens 14 positionierten Boden gerichtet sind, um abhängig von der Bewegungsrichtung den Verdichtungszustand vor oder nach dem Überfahren des Bodens mit dem Bodenverdichter 10 zu erfassen. Eine entsprechende Positionierung ist auch am Nebenrahmen 16 möglich.

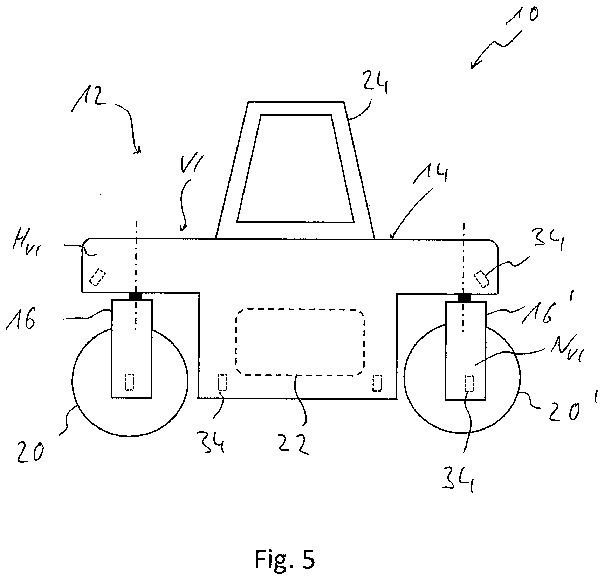

Die Fig. 5 zeigt einen schemelgelenkten Bodenverdichter 10, bei welchem am Hauptrahmen 14 zwei als Lenkschemel ausgebildete Nebenrahmen 16, 16' um jeweilige Lenkachsen A, A' schwenkbar getragen sind. An jedem der als Lenkschemel ausgebildeten Nebenrahmen 16, 16' ist eine Verdichterwalze 20, 20' drehbar getragen. Sowohl in einem Hauptrahmen-Innenvolumenbereich H_{VI}, als auch in jeweiligen Nebenrahmen-Innenvolumenbereichen N_{VI} der Nebenrahmen 16, 16' können eine oder mehrere Bodenradar-Dichtemessanordnungen 34 derart untergebracht sein, dass sie durch jeweilige Messöffnungen hindurch den unter dem Bodenverdichter 10 liegenden Boden erfassen und damit zu Information über dessen Verdichtungszustand bzw. Dichte bereitstellen können.

Es ist abschließend darauf hinzuweisen, dass dann, wenn an den vorangehend beschriebenen Bodenverdichter 10 mehrere Bodenradar-Dichtemessanordnungen 34 vorgesehen sind, diese an beliebigen der vorangehend beschriebenen und in den Figuren dargestellten Positionen untergebracht sein können. Besonders vorteilhaft ist dabei eine Anordnung, bei welcher zumindest zwei Bodenradar-Dichtemessanordnungen 34 in der Bodenverdichter-Längsrichtung L in Abstand zueinander angeordnet sind, beispielsweise so, dass diese in der Bodenverdichter-Längsrichtung L beidseits einer Verdichterwalze 18 bzw. 20 angeordnet sind, oder/und zumindest zwei Bodenradar-Dichtemessanordnungen 34 in der Verdichter-Querrichtung Q in Abstand zueinander angeordnet sind. Beispielsweise könnte bei Verwendung zweier Bodenradar-Dichtemessanordnungen 34 eine dieser Bodenradar-Dichtemessanordnungen 34 im Querträger 26 im Bereich eines Längsendes in der Bodenverdichter-Querrichtung desselben angeordnet sein, während die andere Bodenradar-Dichtemessanordnung 34 im weiteren Querträger 28 im Bereich des anderen Längsendes, also in der Bodenverdichter-Querrichtung Q an oder nahe der anderen Seite des Bodenverdichters 10, angeordnet ist.

## Patentansprüche

1. Bodenverdichter, umfassend wenigstens eine an einem Verdichterrahmen (12) drehbar getragene Verdichterwalze (18, 20) und wenigstens eine in einem Innenvolumenbereich (VI) des Verdichterrahmens (12) angeordnete Bodenradar-Dichtemessanordnung (34).

2. Bodenverdichter nach Anspruch 1,
**dadurch gekennzeichnet, dass** der Verdichterrahmen (12) einen Hauptrahmen (14) und wenigstens einen mit dem Hauptrahmen (14) um eine Lenkachse (A) schwenkbar verbundenen Nebenrahmen (16) umfasst, wobei an dem Hauptrahmen (14) ein zum Antreiben des Bodenverdichters (10) zu betreibendes Antriebsaggregat (22) vorgesehen ist und an dem wenigstens einen Nebenrahmen (16) eine Verdichterwalze (20) drehbar getragen ist.

3. Bodenverdichter nach Anspruch 2,
**dadurch gekennzeichnet, dass** der wenigstens einen Nebenrahmen (16) einen in einer Bodenverdichter-Querrichtung (Q) sich erstreckenden Querträger (26) umfasst, wobei der Nebenrahmen (16) im Bereich des Querträgers (26) mit dem Hauptrahmen (14) schwenkbar verbunden ist.

4. Bodenverdichter nach Anspruch 3,
**dadurch gekennzeichnet, dass** in einem Nebenrahmen-Innenvolumenbereich (N_{VI}) des Querträgers (26) wenigstens eine Bodenradar-Dichtemessanordnung (34) angeordnet ist.

5. Bodenverdichter nach Anspruch 4,
**dadurch gekennzeichnet, dass** der Querträger (26) zum Bereitstellen eines Nebenrahmen-Innenvolumenbereichs (N_{VI}) als Hohlträger ausgebildet ist.

6. Bodenverdichter nach einem der Ansprüche 3-5,
**dadurch gekennzeichnet, dass** der wenigstens eine Nebenrahmen (16) einen in einer Bodenverdichter-Längsrichtung (L) in Abstand zu dem Querträger angeordneten und mit dem Querträger (26)) durch zwei in der Bodenverdichter-Querrichtung (Q) in Abstand zueinander angeordnete und im Wesentlichen in der Bodenverdichter-Längsrichtung (L) sich erstreckende Längsträger (30, 32) verbundenen, im Wesentlichen in der Bodenverdichter-Querrichtung (Q) sich erstreckenden weiteren Querträger (28) umfasst, wobei die Verdichterwalze (20) an den Längsträgern (30, 32) drehbar getragen ist, und dass in einem Nebenrahmen-Innenvolumenbereich (N_{VI}) des weiteren Querträgers (28) oder/und in einem Nebenrahmen-Innenvolumenbereich (N_{VI}) von wenigstens einem Längsträger (30, 32) wenigstens eine Bodenradar-Dichtemessanordnung (34) angeordnet ist.

7. Bodenverdichter nach Anspruch 6,
**dadurch gekennzeichnet, dass** der weitere Querträger (28) oder/und wenigstens einer der Längsträger (30, 32) zum Bereitstellen eines Nebenrahmen-Innenvolumenbereichs (N_{VI}) als Hohlträger ausgebildet ist.

8. Bodenverdichter nach Anspruch 3,
**dadurch gekennzeichnet, dass** zwei als Lenkschemel ausgebildete Nebenrahmen (16, 16') mit dem Hauptrahmen (14) um eine jeweilige Lenkachse (A, A') schwenkbar verbunden sind, und dass in einem Nebenrahmen-Innenvolumenbereich (N_{VI}) von wenigstens einem der als Lenkschemel ausgebildeten Nebenrahmen (16 16') wenigstens eine Bodenradar-Dichtemessanordnung (34) angeordnet ist.

9. Bodenverdichter nach einem der Ansprüche 2-8,
**dadurch gekennzeichnet, dass** der Hauptrahmen (14) einen das Antriebsaggregat (22) und wenigstens eine Bodenradar-Dichtemessanordnung (34) enthaltenden Hauptrahmen-Innenvolumenbereich (H_{VI}) aufweist.

10. Bodenverdichter nach Anspruch 9,
**dadurch gekennzeichnet, dass** in dem Hauptrahmen (14) wenigstens eine vorzugsweise verschließbare Revisionsöffnung (54) vorgesehen ist, und dass auf wenigstens eine, vorzugsweise jede in dem Hauptrahmen-Innenvolumenbereich (H_{VI}) angeordnete Bodenradar-Dichtemessanordnung (34) über wenigstens eine Revisionsöffnung (54) Zugriff besteht.

11. Bodenverdichter nach einem der Ansprüche 1-10,
**dadurch gekennzeichnet, dass** wenigstens eine, vorzugsweise jede Bodenradar-Dichtemessanordnung (34) einen vermittels wenigstens eines elastisch verformbaren Aufhängungselements (48) an dem Verdichterrahmen (12) getragenen Sensorträger (42) und wenigstens einen an dem Sensorträger (42) getragenen Bodenradar-Dichtesensor (44) umfasst.

12. Bodenverdichter nach einem der Ansprüche 1-11,
**dadurch gekennzeichnet, dass** wenigstens eine, vorzugsweise jede Bodenradar-Dichtemessanordnung (34) an einem plattenartigen Trägerbereich (38) des Verdichterrahmens (12) getragen ist.

13. Bodenverdichter nach einem der Ansprüche 1-12,
**dadurch gekennzeichnet, dass** an dem Verdichterrahmen (12) in Zuordnung zu wenigstens einer, vorzugsweise jeder Bodenradar-Dichtemessanordnung (34) eine Messöffnung (40) vorgesehen ist.

14. Bodenverdichter nach einem der Ansprüche 1-13,
**dadurch gekennzeichnet, dass** in Zuordnung zu wenigstens einer, vorzugsweise jeder Verdichterwalze (18, 20) in einer Bodenverdichter-Längsrichtung (L) an jeder Seite der Verdichterwalze (18, 20) wenigstens eine Bodenradar-Dichtemessanordnung (34) vorgesehen ist, oder/und dass wenigstens zwei Bodenradar-Dichtemessanordnungen (34) in einer Bodenverdichter-Querrichtung (Q) in Abstand zueinander angeordnet sind.

## Claims

1. A soil compactor comprising at least one compactor roller (18, 20) rotatably supported on a compactor frame (12) and at least one soil radar density measuring arrangement (34) arranged in an inner volume region (VI) of the compactor frame (12).

2. The soil compactor according to claim 1,
**characterized in that** the compactor frame (12) comprises a main frame (14) and at least one sub-frame (16) pivotably connected to the main frame (14) about a steering axis (A), wherein a drive unit (22) to be operated for driving the soil compactor (10) is provided on the main frame (14) and a compactor roller (20) is rotatably supported on the at least one sub-frame (16).

3. The soil compactor according to claim 2,
**characterized in that** the at least one sub-frame (16) comprises a cross member (26) extending in a soil compactor transverse direction (Q), the sub-frame (16) being pivotably connected to the main frame (14) in the region of the cross member (26).

4. The soil compactor according to claim 3,
**characterized in that** at least one soil radar density measuring arrangement (34) is arranged in a sub-frame inner volume region (N_{VI}) of the cross member (26).

5. The soil compactor according to claim 4,
**characterized in that** the cross member (26) is designed as a hollow member for providing a sub-frame inner volume region (N_{VI}).

6. The soil compactor according to any one of claims 3-5,
**characterized in that** the at least one sub-frame (16) comprises a further cross member (28) arranged in a longitudinal direction (L) of the soil compactor at a distance from the cross member and connected to the cross member (26) by two longitudinal members (30, 32) arranged at a distance from one another in the transverse direction (Q) of the soil compactor and extending substantially in the longitudinal direction (L) of the soil compactor, and which extends substantially in the soil compactor transverse direction (Q), wherein the compactor roller (20) is rotatably supported on the longitudinal members (30, 32), and **in that** at least one soil radar density measuring arrangement (34) is arranged in a sub-frame inner volume region (N_{VI}) of the further cross member (28) or/and in a sub-frame inner volume region (N_{VI}) of at least one longitudinal member (30, 32).

7. The soil compactor according to claim 6,
**characterized in that** the further cross member (28) and/or at least one of the longitudinal members (30, 32) is designed as a hollow member for providing a sub-frame inner volume region (N_{VI}).

8. The soil compactor according to claim 3,
**characterized in that** two sub-frames (16, 16') designed as steering frames are pivotably connected to the main frame (14) about a respective steering axis (A, A'), and **in that** at least one soil radar density measuring arrangement (34) is arranged in a sub-frame inner volume region (N_{VI}) of at least one of the sub-frames (16, 16') designed as steering frames.

9. The soil compactor according to any one of claims 2-8,
**characterized in that** the main frame (14) presents a main frame inner volume region (H_{VI}) containing the drive unit (22) and at least one soil radar density measuring arrangement (34).

10. The soil compactor according to claim 9,
**characterized in that** at least one preferably closable inspection opening (54) is provided in the main frame (14), and **in that** at least one, preferably each, soil radar density measuring arrangement (34) arranged in the main frame inner volume region (H_{VI}) is accessible via at least one inspection opening (54).

11. The soil compactor according to any one of claims 1-10,
**characterized in that** at least one, preferably each soil radar density measuring arrangement (34) comprises a sensor carrier (42) carried on the compactor frame (12) by means of at least one elastically deformable suspension element (48) and at least one soil radar density sensor (44) carried on the sensor carrier (42).

12. The soil compactor according to any one of claims 1-11,
**characterized in that** at least one, preferably each, soil radar density measuring arrangement (34) is supported on a plate-like carrier region (38) of the compactor frame (12).

13. The soil compactor according to any one of claims 1-12,
**characterized in that** a measuring opening (40) is provided on the compactor frame (12) in association with at least one, preferably each, soil radar density measuring arrangement (34).

14. The soil compactor according to any one of claims 1-13,
**characterized in that** at least one soil radar density measuring arrangement (34) is provided on each side of the compactor roller (18, 20) in a longitudinal soil compactor longitudinal direction (L) in association with at least one, preferably each, compactor roller (18, 20), or/and **in that** at least two soil radar density measuring arrangements (34) are arranged at a distance from one another in a soil compactor transverse direction (Q).

## Revendications

1. Un compacteur de sol comprenant au moins un rouleau de compacteur (18, 20) monté de manière rotative sur un châssis de compacteur (12) et au moins un dispositif de mesure de densité de radar de sol (34) disposé dans une zone de volume interne (VI) du châssis de compacteur (12).

2. Compacteur de sol selon la revendication 1,
**caractérisé en ce que** le châssis de compacteur (12) comprend un châssis principal (14) et au moins un châssis secondaire (16) relié de manière pivotante au châssis principal (14) autour d'un axe de direction (A), dans lequel une unité d'entraînement (22) destinée à être actionnée pour entraîner le compacteur de sol (10) est prévue sur le châssis principal (14) et un rouleau de compacteur (20) est supporté de manière rotative sur ledit au moins un châssis secondaire (16).

3. Le compacteur de sol selon la revendication 2,
**caractérisé en ce que** ledit au moins un châssis secondaire (16) comprend une traverse (26) s'étendant dans une direction transversale (Q) du compacteur de sol, le châssis secondaire (16) étant relié de manière pivotante au châssis principal (14) dans la région de la traverse (26).

4. Le compacteur de sol selon la revendication 3,
**caractérisé en ce qu'**au moins un dispositif de mesure de densité de radar de sol (34) est disposé dans une zone de volume interne de châssis secondaire (N_{VI}) de la traverse (26).

5. Le compacteur de sol selon la revendication 4,
**caractérisé en ce que** la traverse (26) est conçue comme un élément creux afin de former une zone de volume interne de châssis secondaire (N_{VI}).

6. Compacteur de sol selon l'une quelconque des revendications 3 à 5, **caractérisé en ce que** ledit au moins un châssis secondaire (16) comprend une autre traverse (28) disposée dans une direction longitudinale (L) du compacteur de sol à distance de la traverse et reliée à la traverse (26) par deux éléments longitudinaux (30, 32) disposés à distance l'un de l'autre dans la direction transversale (Q) du compacteur de sol et s'étendant sensiblement dans la direction longitudinale (L) du compacteur de sol, et qui s'étend sensiblement dans la direction transversale (Q) du compacteur de sol, dans lequel le rouleau de compacteur (20) est supporté de manière rotative sur les éléments longitudinaux (30, 32), et **en ce qu'**au moins un dispositif de mesure de densité de radar de sol (34) est disposé dans une zone de volume interne de châssis secondaire (N_{VI}) de la traverse supplémentaire (28) et/ou dans une zone de volume interne de châssis secondaire (N_{VI}) d'au moins un élément longitudinal (30, 32).

7. Le compacteur de sol selon la revendication 6,
**caractérisé en ce que** la traverse supplémentaire (28) et/ou au moins l'un des éléments longitudinaux (30, 32) est conçu comme un élément creux pour former une zone de volume interne de châssis secondaire (N_{VI}).

8. Le compacteur de sol selon la revendication 3,
**caractérisé en ce que** deux châssis secondaires (16, 16') conçus comme des châssis de direction sont reliés de manière pivotante au châssis principal (14) autour d'un axe de direction respectif (A, A'), et **en ce qu'**au moins un dispositif de mesure de densité de radar de sol (34) est disposé dans une zone de volume interne de châssis secondaire (N_{VI}) d'au moins l'un des châssis secondaires (16, 16') conçus comme châssis de direction.

9. Le compacteur de sol selon l'une quelconque des revendications 2 à 8, **caractérisé en ce que** le châssis principal (14) présente une zone de volume interne (H_{VI}) contenant l'unité d'entraînement (22) et au moins un dispositif de mesure de densité de radar de sol (34).

10. Le compacteur de sol selon la revendication 9,
**caractérisé en ce qu'**au moins une ouverture d'inspection (54), de préférence pouvant être fermée, est prévue dans le châssis principal (14), et **en ce qu'**au moins un, de préférence chaque, dispositif de mesure de densité de radar de sol (34) disposé dans la zone de volume interne de châssis principal (H_{VI}) est accessible via au moins une ouverture d'inspection (54).

11. Le compacteur de sol selon l'une quelconque des revendications 1 à 10, **caractérisé en ce qu'**au moins un, de préférence chaque dispositif de mesure de densité de radar de sol (34) comprend un support de capteur (42) porté sur le châssis de compacteur (12) au moyen d'au moins un élément de suspension élastiquement déformable (48) et au moins un capteur de densité de radar de sol (44) supporté sur le support de capteur (42).

12. Le compacteur de sol selon l'une quelconque des revendications 1 à 11, **caractérisé en ce qu'**au moins un, de préférence chaque, dispositif de mesure de densité de radar de sol (34) est supporté sur une zone de support en forme de plaque (38) du châssis de compacteur (12).

13. Le compacteur de sol selon l'une quelconque des revendications 1 à 12, **caractérisé en ce qu'**une ouverture de mesure (40) est prévue sur le châssis de compacteur (12) en association avec au moins un, de préférence chaque, dispositif de mesure de densité de radar de sol (34).

14. Le compacteur de sol selon l'une quelconque des revendications 1 à 13, **caractérisé en ce qu'**au moins un dispositif de mesure de densité de radar de sol (34) est prévu de chaque côté du rouleau de compacteur (18, 20) dans une direction longitudinale (L) du compacteur de sol, en association avec au moins un, de préférence chaque, rouleau de compacteur (18, 20), ou/et **en ce qu'**au moins deux dispositifs de mesure de densité de radar de sol (34) sont disposés à distance l'un de l'autre dans une direction transversale (Q) du compacteur de sol.
